# EUROPEAN PATENT SPECIFICATION

(11) **EP 1 838 275 B1**
(45) Date of publication and mention of the grant of the patent: **09.11.2016**
(21) Application number: 05824886.5
(22) Date of filing: 18.11.2005
(51) Int. Cl.: A61K 8/97, A61Q 11/00

(54) **OXIDATION RESISTANT DENTIFRICE COMPOSITIONS**
OXIDATIONSBESTÄNDIGE ZAHNPFLEGEZUSAMMENSETZUNGEN
FORMULES DE DENTIFRICE RÉSISTANTES À L'OXYDATION

(30) Priority: 29.12.2004 US 25577
(43) Date of publication of application: 03.10.2007
(73) Proprietor: Colgate-Palmolive Company, New York, NY 10022 (US)
(72) Inventor: ARVANITIDOU, Evangelia, S., Princeton, New Jersey 08540 (US); PRENCIPE, Michael, West Windsor, New Jersey 08550 (US)
(74) Representative: Jenkins, Peter David
(86) International application number: PCT/US2005/042163
(87) International publication number: WO 2006/071404

(56) References cited:
- WO-A-2004/041234
- WO-A-2005/025527
- DE-A1- 4 221 103
- US-B1- 6 645 472
- DATABASE CA [Online] CHEMICAL ABSTRACTS SERVICE, COLUMBUS, OHIO, US; MARUYAMA, MASATATSU ET AL: "Discoloration-free anticaries dentifrice compositions containing glucosyltransferase inhibitors and sugar alcohols" XP002373139 retrieved from STN Database accession no. 2000:748806 & JP 2000 297022 A2 (LION CORP., JAPAN) 24 October 2000 (2000-10-24)
- DATABASE CA [Online] CHEMICAL ABSTRACTS SERVICE, COLUMBUS, OHIO, US; KONDO, MITSUO: "Prevention of discoloration in polyphenol-containing cosmetics by copper-porphyrin complexes and organic reducing agents" XP002373140 retrieved from STN Database accession no. 1989:121132 & JP 63 145213 A2 (KANEBO, LTD., JAPAN) 17 June 1988 (1988-06-17)
- DATABASE CA [Online] CHEMICAL ABSTRACTS SERVICE, COLUMBUS, OHIO, US; "Color stabilization by titanium dioxide in tooth pastes" XP002373141 retrieved from STN Database accession no. 1983:563856 & JP 58 118508 A2 (LION CORP., JAPAN) 14 July 1983 (1983-07-14)
- PATENT ABSTRACTS OF JAPAN vol. 011, no. 394 (C-465), 23 December 1987 (1987-12-23) & JP 62 155210 A (LION CORP), 10 July 1987 (1987-07-10)

## Description

### INTRODUCTION

The invention relates to dentifrices compositions containing antioxidant active ingredients. In particular, the invention relates to non-aqueous dentifrices showing oxidation stability.

Formulated dentifrices such as tooth pastes and gels contain a number of functional and active ingredients, each of which contribute to one or a number of desirable properties. Properly formulated dentifrices are suitable for regular use to promote oral health. Functional additives include abrasive materials that remove stains and other materials from tooth surfaces, foaming agents that disperse other ingredients and provide for delivery of the active and functional materials to the oral surfaces, and tartar control agents to prevent the formation of calculus on tooth surfaces, as well as aesthetic functional ingredients such as flavors and pigments. Active ingredients include anticaries agents that provide a source of fluoride ion upon use. Various compositions also contain compounds or components with antibacterial properties, for example to reduce the formation of plaque on the surfaces. Further active ingredients include those with anti-inflammatory properties for prophylaxis and treatment of conditions such as gingivitis.

Antioxidants have been proposed as ingredients of oral care compositions such as dentifrices. The antioxidants may act as a stabilizing agent by protecting a readily oxidizable ingredient from degradation. In addition, components with antioxidant activity can also act as agents to enhance oral and/or whole body health.

Ideally, dentifrice compositions should be stable during manufacture, shipping, and use by the consumer. An important function of antioxidant components of dentifrice compositions is to prevent deterioration of the dentifrice. In various aspects, such deterioration could manifest itself as a loss of efficacy in various active ingredients or a loss in the esthetic properties of the dentifrice, such as taste and appearance.

In one mode of operation, antioxidant components act by themselves being sacrificially oxidized in order that other components of the composition can be protected. Unsightly oxidation products of the antioxidant additives in a dentifrice composition can lead to undesirable coloring or browning of the compositions. Such discoloration can be noticeable especially in compositions that are lightly colored or are pigmented to be white.

It is thus always desirable to provide toothpaste and other dentifrice compositions with a wide variety of active and functional materials. It is further desirable to provide antioxidant properties to the dentifrice composition, without leading to undesirable side affects such as discoloration.

DE-A-4221103 discloses oral care compositions containing herbal extracts.

US-A-6,645,472 discloses anhydrous tooth and gum powdered dentifrice formulations including green tea extract.

JP-A-2000-2970222 discloses an oral cavity composition including a polyphenol-containing plant extract and a sugar alcohol.

JP-A-63-145213 discloses the prevention of change of the colour of a dissolved polyphenol by blending with a porphyrin metallic complex containing copper as a ligand metal and an organic reducing agent.

JP-A-58-118508 discloses a dentifrice composition containing a coloured vegetable effective fraction and titanium dioxide as a discolouration inhibitor.

JP-A-62-155210 discloses an oral cavity composition containing an extract of roots of a plant of the family Labiatae adjusted to pH 4-8.

WO-A-2004/041234 discloses an anhydrous composition comprising an antioxidant comprising over 40% by weight of hydrolysable tannins having a molecular weight of less than 1000.

### SUMMARY

The present invention provides an oral composition according to claim 1 and a method for reducing the extent of discolouration in a toothpaste according to claim 9. According to one aspect of the invention, dentifrice compositions are provided that contain various plant components such as flavonoids, catechins, and polyphenols. Sources of the plant components include a variety of plant extracts such as rosemary extract and green tea extract. Toothpastes and other dentifrices are provided that contain the components yet are resistant to discoloration from oxidation.

Thus, in one aspect the invention provides low water tooth pastes containing a variety of plant extracts. The invention provides dentifrice compositions containing humectants, abrasive compounds, and a variety of plant extracts, such as rosemary and green tea extracts, along with an additional antioxidant component. Antioxidants include stannous compounds, sodium metabisulfite, BHT, ammonium sulfate, and potassium stannate. The composition of the invention can be used in methods for promoting oral health of a subject animal comprising applying a composition as discussed above to the oral surfaces of the animal.

### DESCRIPTION

The headings (such as "Introduction" and "Summary,") used herein are intended only for general organization of topics within the disclosure of the invention, and are not intended to limit the disclosure of the invention or any aspect thereof. In particular, subject matter disclosed in the "Introduction" may include aspects of technology within the scope of the invention, and may not constitute a recitation of prior art. Subject matter disclosed in the "Summary" is not an exhaustive or complete disclosure of the entire scope of the invention or any embodiments thereof. Similarly, subpart headings in the Description are given for convenience of the reader, and are not a representation that information on the topic is to be found exclusively at the heading.

The description and specific examples, while indicating embodiments of the invention, are intended for purposes of illustration only and are not intended to limit the scope of the invention. Moreover, recitation of multiple embodiments having stated features is not intended to exclude other embodiments having additional features, or other embodiments incorporating different combinations of the stated features. Specific Examples are provided for illustrative purposes of how to make, use and practice the compositions and methods of this invention and, unless explicitly stated otherwise, are not intended to be a representation that given embodiments of this invention have, or have not, been made or tested.

As used herein, the words "preferred" and "preferably" refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, the word "include," and its variants, is intended to be non-limiting, such that recitation of items in a list is not to the exclusion of other like items that may also be useful in the materials, compositions, devices, and methods of this invention.

An "orally acceptable amount" of a compound is an amount that is not harmful to a mammal when a composition containing such amount is retained in the mouth, without swallowing, for a period sufficient to permit application to an oral surface as provided herein. In general, such amount of the compound is not harmful even if the composition is unintentionally swallowed.

"Antibacterial activity" herein means activity as determined by any generally accepted *in vitro* or *in vivo* antibacterial assay or test.

"Anti-inflammatory activity" herein means activity as determined by any generally accepted *in vitro* or *in vivo* assay or test, for example an assay or test for inhibition of prostaglandin production or cyclooxygenase activity.

"Antioxidant activity" herein means activity as determined by any generally accepted in vitro or in vivo antioxidant assay or test.

An "oral surface" herein encompasses any soft or hard surface within the mouth including surfaces of the tongue, hard and soft palate, buccal mucosa, gums and dental surfaces. A "dental surface" herein is a surface of a natural tooth or a hard surface of artificial dentition including a crown, cap, filling, bridge, denture, dental implant and the like.

A step of "applying" a composition to an oral surface herein encompasses any procedure that results in the composition contacting the surface, including irrigating, rinsing, spraying, wiping, rubbing, brushing, painting, flossing, placement of a film or strip on the surface, implanting and chewing.

The term "inhabiting" herein with respect to a condition such as inflammation in an oral tissue encompasses prevention, suppression, reduction in extent or severity, or amelioration of the condition.

The invention provides an oral or dentifrice composition, such as a tooth paste or gel according to claim 1.

The tooth pastes or dentifrices can be formulated with additional active and functional materials such as anticalculus systems, antibacterial compositions, anticaries agents, and the like.

In an embodiment, the oral composition of the invention provides a tooth paste or gel composition As before, the compositions are optionally formulated with other functional and active materials such as, without limitation, anticalculus, anticaries, antibacterial, and anti-inflammatory agents.

The composition of the invention may be used in methods for promoting oral health of a subject animal comprising applying any of the toothpaste or dentifrice compositions described above to the oral surfaces of the animal. The methods may be for veterinary as well as human use.

The methods are based in part of the discovery that discoloration of dentifrice compositions including flavonoid compounds can be reduced or mitigated by formulating the composition to contain less than 10% by weight water, and by adding certain antioxidant materials to the formulation. Thus, the invention provides methods for reducing the extent of discoloration in a toothpaste where the toothpaste contains plant comprising flavonoids which tend to oxidize and discolor the toothpaste composition over time. The methods comprise formulating the toothpaste to contain less than 10% by weight water and adding to the toothpaste an amount effective to reduce discoloration of an antioxidant selected from group exemplified by stannous compounds; stannate compounds; ammonium sulfate; phenol antioxidants such as BHT; hydroquinones such as BHA; and metabisulfite salts such as sodium metabisulfite and potassium metabisulfite. In various embodiments, the compositions used in the method contain less than 8% by weight water or less than 6% by weight water. The flavonoid of the dentifrice compositions are generally derived from a variety of plant extracts, such as without limitation rosemary extract and green tea extract. The compositions used in the methods are optionally formulated to contain other functional and active materials such as anticalculus agents, antibacterial agents, anticaries ingredients, and the like.

A number of plant extracts can be used in the dentifrice composition to provide a source of flavonoid components. Generally, the plant extracts contain a number of oxidizable species that tend to produce color over time when formulated into a composition such as a tooth paste or gel. These are called plant-derivable compounds because they occur naturally in plants and can be isolated from them. Non-limiting examples of plants from which extracts can be provided include *Rosmarinus officinalis* (rosemary), *Camellia sinensis* (green tea), *Origanum vulgare* (oregano), *Scutellaria baicalensis* (baikal skullcap), *Vitis vinifera* (grape seed and grape skin), and other *Scutellaria* species such as *S. orthocalyx* and *S. lateriflora.* Such plant extracts are generally prepared by extraction of leaves or other components of the plants by known methods. Non-limiting examples include extraction with a variety of solvents such as water, alcohols (especially ethanol), supercritical fluids such as carbon dioxide, and chlorofluorocarbons. Solvent soluble components are thus extracted from various plant materials by various known methods. Many plant extracts are commercially available. Suppliers include Sabinsa Corporation and Indena S.p.A., and Guangzhou-Honsea Sunshine BST. Oral composition contain from about 0.001 to about 5.0 weight percent of plant extract or of plant-derivable compounds. In various embodiments, compounds such as flavonoids, catechins, polyphenols, and tannins are added directly to oral compositions of the invention. Suitable levels are from about 0.001 to about 5 percent by weight of the oral composition, for example from 0.01 to 5%, 0.01 to 1%, 0.05 to 2%, 0.05 to 2%, 0.1 to 1%, 0.1 to 0.5%, and 0.1 to 0.3%. The compounds make up less than 100% of the extract, so relatively higher levels of added extract are needed to achieve a certain level of the compounds. However, in various embodiments, the compositions Contain plant extracts at the levels listed above.

Antioxidants for use in dentifrice compositions of the invention include stannous compounds. Stannous compounds include organic and inorganic sources capable of releasing stannous ion. Non-limiting inorganic sources include stannous chloride, fluoride, sulfate, phosphate, metaphosphate, and pyrophosphate. Organic source include, without limitation, stannous acetate, tartrate, citrate, malate, malonate, gluconate, and oxalate. Further examples of antioxidants include stannate materials such as potassium stannate; ammonium sulfate; phenolic antioxidants such as BHT; hydrioquinone antioxidants such as BHA; and sulfur containing antioxidants such as sodium metabisulfite.

Without limitation, treatment levels range from about 0.01% to about 1% of the antioxidant, based on the total weight of the dentifrice composition. For the stannous and stannate antioxidant, preferred ranges include from about 0.05% to about 0.5%, preferably from about 0.1% to about 0.5%.

In various embodiments, the dentifrice compositions contain relatively low amounts of water. In many aspects, the dentifrice compositions contain less water than typical of current commercial formulations. The compositions of the invention contain less than 6% by weight water. The total amount of water in the dentifrice compositions includes contributions from water intentionally added as a component and water present as a byproduct or solvent for various other components. In various embodiments, the dentifrice compositions are formulated without adding water as a separate component. The resulting water content of the dentifrice composition is then derived from the residual water present as solvent or byproduct in the various components. As discussed above, a formulated dentifrice generally contains 6% or less by water.

Flavonoids are members of a large category of plant products that derive from γ-pyrone. In one aspect, the compounds are derived either from 2-phenylbenzopyrone (1) or 3-phenylbenzopyrone (2): 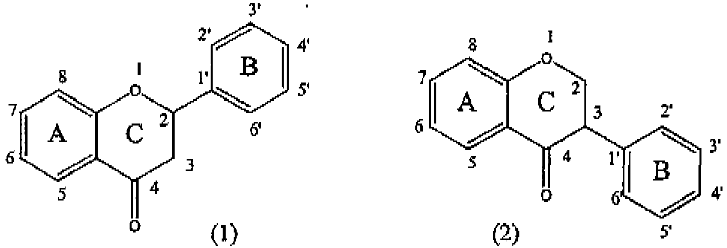 Individual flavonoids can be classified into various groups, which differ among themselves by the oxidation level and/or the substitution pattern on the heterocyclic ring (ring C). Among the groups are chalcones, flavanones, isoflavanones, flavones, anthocyanidins (flavylium cations), flavan-3-ols (catechins), flavan-3,4-diols (proanthocyanidins), biflavonoids and oligomeric flavonoids, isoflavonoids, aurones, and free beta ring flavonoids. Many of these groups are illustrated here.

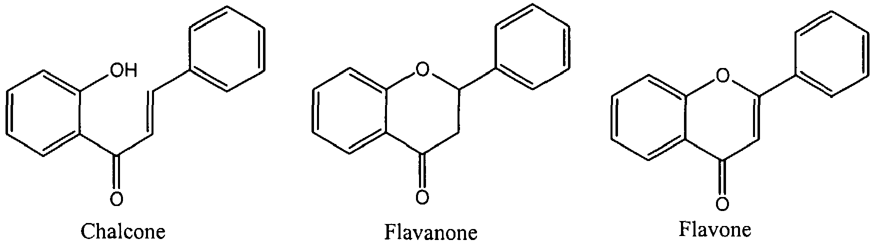

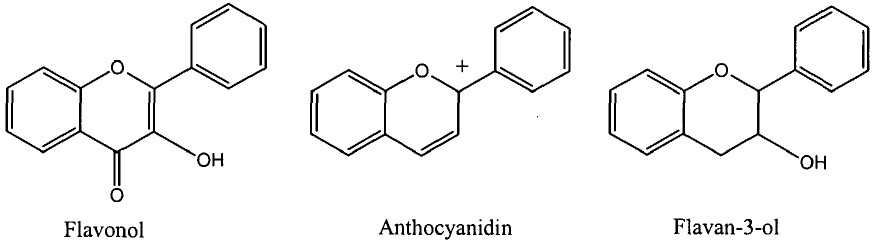

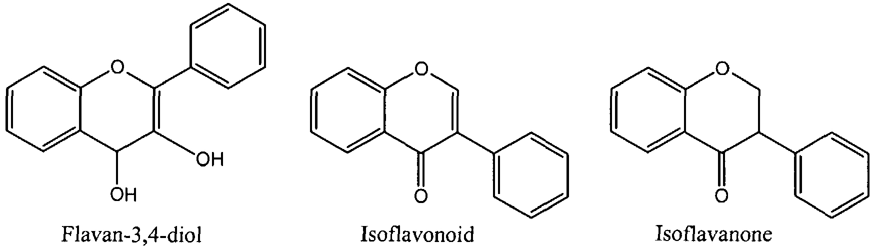

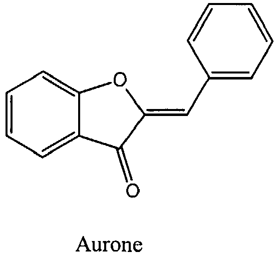

There are many hundreds or thousands of flavonoid compounds that have been isolated and identified from plants. Flavonoids are components of plant extracts, since they are soluble in the solvents used to prepare the extracts. The basic classes of flavonoids differ according to the oxidation state of ring B and the site of attachment of ring C, as indicated above. Within a class, individual flavonoids differ by the number and position of substituent groups on positions 1 - 8 and 1' - 6'. The most common constituents include hydroxy, methoxy, and sugars. Among the flavones and flavonones, positions 3', 4', 5, and 7 are the most commonly substituted, while for the flavonols, anthocyanidins, and flavan-3-ols, position 3 is often additionally substituted. The number of substituents varies widely, and can range from zero to eight, with four to six being most common.

Flavonoids occur frequently in plants as glycosides, with hexoses such as glucose, galactose, and rhamnose and pentoses such as arabinose and xylose being the most common. For flavanones and flavones the 7-hydroxyl is most commonly glycosylated; for flavonols it the 3- and 7-hydroxyls, while for the anthocyanins it is the 3- and 5-hydroxyls. The sugars attached to the flavonoids in this way are often themselves esterified, for example with p-coumaric, caffeic, ferulic, and sinapic acid.

Another class of flavonoids is the free B-ring flavones and flavanols, which have no substituent groups on the B-ring. Free B-ring flavonoids have been reported to have diverse biological activity. Sources include the roots of *Scutellaria* species such as *S. baicalensis, S. lateriflora,* and *S. orthocalyx.*

Depending on the basic skeleton, flavonoids can be detected by their absorption in the visible between about 330 and 370 nanometers. Aromatic esters such as coumaric, caffeic, ferulic, and sinapic are usually marked by an additional band between about 307 and 340 nanometers.

In various embodiments, flavonoid compounds as described above represent minor components of the plant extracts. For example, rosemary extract contains major amounts of carnosic, ursolic, and oleanolic acid and lesser amounts of flavonoids, while the extract of *Scutellaria baicalensis* contains baicilin. As a result, even though many flavonoids are colored as shown by their absorption in the visible region, their treat levels in preferred embodiments are sufficiently low that pigmenting is not unacceptably interfered with. For example, white dentifrices and tooth pastes can be formulated by adding white pigments such as titanium dioxide that tend to mask any slight amount of color in the flavonoids present at low levels. Alternatively, compositions can be pigmented with colored pigments that are compatible with or even augment any natural color given by the flavonoids.

Polyphenols are a class of plant-derivable compounds characterized by the presence in the molecular structure of two or more phenyl rings that contain a hydroxy substituent. Depending on the substitution pattern of the flavonoids shown above, many of the flavonoids are also polyphenols.

Tannins are a broad but well defined category of plant-derivable compounds. Tannins are polyphenolic secondary metabolites of higher plants. Representative of one class of tannins are the galloyl esters and their derivatives, where galloyl moieties are attached to a variety of polyol, catechin, and triterpenoid cores (gallotannins, ellagitannins, and complex tannins, respectively). Another class includes complex tannins that are oligomeric and polymeric proanthocyanadins; these can have various interflavanyl coupling and substitution patterns. Tannins are described in Natural Products Reports, vol. 18, p. 641-649 (Royal Society of Chemistry 2001).

The compounds are plant-derivable in that they can be isolated from the leaves and other parts of higher plants such as those described above. The compounds are added in the form of plant extracts. These are complex mixtures of solvent soluble components which, as described above, are isolated by solvent extraction of parts of the plants.

In dentifrice compositions with a relatively high amount of water, and in various embodiments where the compositions lack suitable levels of antioxidants, oxidation of the plant-derivable compounds over time can lead to an undesirable darkening or browning even of pigmented dentifrices, which would be a disadvantage or drawback in the eyes of many consumers. In various embodiments, compositions of the invention overcome such a drawback.

In addition to the plant derivable compounds and the antioxidant components, the oral or dentifrice compositions of the invention contain orally acceptable amounts of a number of other active and non-active materials that are commonly found in tooth pastes and the like. In various embodiments, advantages are provided by certain combinations of these otherwise conventional components with each other and with the plant extracts and plant-derivable compounds described here.

Dentifrice compositions contain at least one humectant, useful for example to prevent hardening of a toothpaste upon exposure to air. Any orally acceptable humectant can be used, including without limitation polyhydric alcohols such as glycerin, propylene glycol, sorbitol, xylitol and low molecular weight polyethylene glycol (PEG). Most humectants also function as sweeteners. One or more humectants are present in a total amount of about 1% to about 70%, for example about 1% to about 50%, about 2% to about 25%, or about 5% to about 15% by weight of the composition.

The compositions of the invention comprises at least one
abrasive, useful for example as a polishing agent. Any orally acceptable abrasive can be used, but type, fineness (particle size) and amount of abrasive should be selected so that tooth enamel is not excessively abraded in normal use of the composition. Suitable abrasives include without limitation silica, for example in the form of silica gel, hydrated silica or precipitated silica, alumina, insoluble phosphates, calcium carbonate, resinous abrasives such as urea-formaldehyde condensation products and the like. Among insoluble phosphates useful as abrasives are orthophosphates, polymetaphosphates and pyrophosphates. Illustrative examples are dicalcium orthophosphate dihydrate, calcium pyrophosphate, *β-*calcium pyrophosphate, tricalcium phosphate, calcium polymetaphosphate and insoluble sodium polymetaphosphate. One or more abrasives are present in an abrasive effective total amount, typically about 5% to about 70%, for example about 10% to about 50% or about 15% to about 30% by weight of the composition. Average particle size of an abrasive, if present, is generally about 0.1 to about 30 *µ*m, for example about 1 to about 20 *µ*m or about 5 to about 15 *µ*m.

In another embodiment the compositions comprise an orally acceptable anticalculus agent. One or more such agents can be present. Suitable anticalculus agents include without limitation phosphates and polyphosphates (for example pyrophosphates), polyaminopropanesulfonic acid (AMPS), zinc citrate trihydrate, polypeptides such as polyaspartic and polyglutamic acids, polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and salts of any of these agents, for example their alkali metal and ammonium salts. Useful inorganic phosphate and polyphosphate salts illustratively include monobasic, dibasic and tribasic sodium phosphates, sodium tripolyphosphate (STPP), tetrapolyphosphate, mono-, di-, trisodium pyrophosphates, tetrasodium pyrophosphates(TSPP), disodium dihydrogen pyrophosphate, sodium trimetaphosphate, sodium hexametaphosphate and the like, wherein sodium can optionally be replaced by potassium or ammonium. Other useful anticalculus agents include polycarboxylate polymers. These include polymers and copolymers of carboxylic monomers such as acrylic acid, methacrylic acid, and maleic anhydride. Non-limiting examples include polyvinyl methyl ether/maleic anhydride (PVME/MA) copolymers, such as those available under the Gantrez™ brand from ISP, Wayne, NJ. In various embodiments, the anticalculus agent is made of phosphate compounds such as STPP and TSPP, and polycarboxylate polymer such as the Gantrez® types as an anionic carboxylate component. Still other useful anticalculus agents include sequestering agents including hydroxycarboxylic acids such as citric, fumaric, malic, glutaric and oxalic acids and salts thereof, and aminopolycarboxylic acids such as ethylenediaminetetraacetic acid (EDTA). One or more anticalculus agents are optionally present in the composition in an anticalculus effective total amount, typically about 0.01% to about 50%, for example about 0.05% to about 25% or about 0.1% to about 15% by weight.

In another embodiment the composition comprises an orally acceptable anticaries agent, which is a source of fluoride ions. One or more such sources can be present. Suitable sources of fluoride ions include fluoride, monofluorophosphate and fluorosilicate salts. Another non-limiting example is amine fluorides, including olaflur (N'-octadecyltrimethylendiamine-N,N,N'- tris(2-ethanol)-dihydrofluoride). Any such source that is orally acceptable can be used, including without limitation alkali metal (e.g., potassium, sodium), ammonium, stannous and indium salts and the like. Water-soluble fluoride-releasing salts are typically used. One or more fluoride-releasing salts are optionally present in an amount providing a total of about 100 to about 20,000 ppm, about 200 to about 5,000 ppm, or about 500 to about 2,500 ppm, fluoride ions. Where sodium fluoride is the sole fluoride-releasing salt present, illustratively an amount of about 0.01% to about 5%, about 0.05% to about 1% or about 0.1% to about 0.5%, sodium fluoride by weight can be present in the composition.

In another embodiment the composition comprises an orally acceptable antimicrobial (e.g., antibacterial) agent. One or more such agents can be present. They can be phenolic compounds or other antibacterial compounds.

Non-limiting examples of antibacterial phenolic compounds include 4-allylcatechol, *p*-hydroxybenzoic acid esters including benzylparaben, butylparaben, ethylparaben, methylparaben and propylparaben, 2-benzylphenol, butylated hydroxyanisole, butylated hydroxytoluene, capsaicin, carvacrol, creosol, eugenol, guaiacol, halogenated bisphenolics including hexachlorophene and bromochlorophene, 4-hexylresorcinol, 8-hydroxyquinoline and salts thereof, salicylic acid esters including menthyl salicylate, methyl salicylate and phenyl salicylate, phenol, pyrocatechol, salicylanilide, thymol, triclosan and triclosan monophosphate.

The antibacterial phenolic compound is in one aspect a halogenated diphenylether, for example triclosan, triclosan monophosphate or 2,2'-dihydroxy-5,5'-dibromodiphenylether.

The antibacterial phenolic compound is present in an amount to provide sufficient antibacterial properties to the compositions, for example about 0.01% to about 10% by weight. Illustratively the total concentration of the phenolic compound in a toothpaste or gel dentifrice can be about 0.05% to about 5%, for example about 0.1 % to about 2%, about 0.2% to about 1% or about 0.25% to about 0.5%.

Other suitable antibacterial agents include without limitation copper (II) compounds such as copper (II) chloride, fluoride, sulfate and hydroxide, zinc ion sources such as zinc acetate, zinc citrate, zinc gluconate, zinc glycinate, zinc oxide, zinc sulfate and sodium zinc citrate, phthalic acid and salts thereof such as magnesium monopotassium phthalate, hexetidine, octenidine, sanguinarine, benzalkonium chloride, domiphen bromide, alkylpyridinium chlorides such as cetylpyridinium chloride (CPC) (including combinations of CPC with zinc and/or enzymes), tetradecylpyridinium chloride and N-tetradecyl-4-ethylpyridinium chloride, iodine, sulfonamides, bisbiguanides such as alexidine, chlorhexidine and chlorhexidine digluconate, piperidino derivatives such as delmopinol and octapinol, magnolia extract, grapeseed extract, menthol, geraniol, citral, eucalyptol, antibiotics such as augmentin, amoxicillin, tetracycline, doxycycline, minocycline, metronidazole, neomycin, kanamycin and clindamycin, and the like. A further illustrative list of useful antibacterial agents is provided in U.S. Patent No. 5,776,435 to Gaffar et al.*,* incorporated herein by reference. The antimicrobial agents are optionally present in an antimicrobial effective total amount, typically about 0.05% to about 10%, for example about 0.1% to about 3% by weight, of the composition.

In a still further embodiment a composition of the invention comprises at least one surfactant, useful for example to compatibilize other components of the composition and thereby provide enhanced stability, to help in cleaning the dental surface through detergency, and to provide foam upon agitation, e.g., during brushing with a dentifrice composition of the invention. Any orally acceptable surfactant, most of which are anionic, nonionic or amphoteric, can be used. Suitable anionic surfactants include without limitation water-soluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate, sodium coconut monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of C₈₋₂₀ aliphatic secondary and tertiary amines having an anionic group such as carboxylate, sulfate, sulfonate, phosphate or phosphonate. A suitable example is cocoamidopropyl betaine. One or more surfactants are optionally present in a total amount of about 0.01% to about 10%, for example about 0.05% to about 5% or about 0.1% to about 2% by weight of the composition.

The compositions optionally contain other active ingredients. Non-limiting examples include desensitizing agents, stannous ion sources, zinc ion sources, sialagogues, breath-freshening agents, antiplaque agents, anti-inflammatory agents additional to any anti-inflammatory phenolic compound present, periodontal agents, anti-gingivitis agents, analgesics and nutrients. Actives should be selected for compatibility with each other and with other ingredients of the composition.

The oral compositions optionally contain one or more other non-active ingredients. Non-limiting examples include diluents, , bicarbonate salts, pH modifying agents, foam modulators, thickening agents, viscosity modifiers, pigmenting agents, sweeteners, flavorants and colorants. Tooth pastes, tooth gels, and similar dentifrice compositions are formulated with these and optionally other additives according to known principles.

The invention has been described above with respect to several preferred embodiments. Further non-limiting description is provided in the Examples that follow.

### EXAMPLES

### Example 1 - Base Formula

A tooth paste composition is provided that contains plant extract as shown in Table 1. Examples of plant extracts include rosemary, green tea, oregano, grape seed, grape skin, *S*. *baicalensis, S*. *lateriflora,* and *S*. *orthocalyx.*

**Table 1**

| **Ingredients** | **Percent (%)** |
|---|---|
| Synthetic Glycerin (99%) | 20 |
| Sorbitol (70%) | 20.85 |
| Sodium CMC | 1.1 |
| Iota Carrageenan | 0.4 |
| Sodium Saccharin | 0.3 |
| Sodium Fluoride | 0.243 |
| Titanium Dioxide | 0.5 |
| Abrasive Silica (Zeodent 115) | 1.520 |
| Thickener Silica (Zeodent 165) | 1.5 |
| Flavor | 1 |
| Sodium lauryl Sulfate | 1.5 |
| plant extract | 0.3 |
| Water | Rest |

### Example 2 - Addition of Reducing Agents

The base formula containing plant extracts of Example 1 is exposed to accelerated aging at 37°C or 50°C for periods of three days or longer. In addition, the tooth paste compositions contain the antioxidant noted in Table 2. The color is subjectively evaluated on a scale from 1 to 5. On the scale, a rating of 5 corresponds to no observable discoloration; a rating of 1 is given for formulations showing the highest degree of discoloration in the aging tests.

**Table 2**

| **Formula** | **Color evaluation*** |
|---|---|
| Base formula | 1.0 |
| Base Formula + 0.1% Stannous Chloride | 3.0 |
| Base Formula + 0.3% Stannous Chloride | 4.0 |
| Base Formula +0.1% Stannous Fluoride | 3.0 |
| Base Formula + 0.3% Stannous Fluoride | 4.5 |
| Base Formula + 0.2% Potassium Stannate trihydrate | 2.5 |
| Base Formula + 0.2% Ammonium Sulfate | 3.5 |
| Base Formula + 0.3% BHT | 4.5 |
| Base Formula + 0.114% Sodium meta-bisulfite | 5.0 |

| | |
|---|---|
| * Scale: 1: Worst 5: Best | |

### Example 3 - "Low" Water Formulations

A formulation containing plant extract and no added water is prepared as in Table 3. Plant extracts are those of Example 1, and the rating scale is that of Example 2.

**Table 3**

| **Ingredients** | **Formula A%** | **Formula B%** |
|---|---|---|
| Polyethylene Glycol 300 | 7 | 7 |
| Propylene Glycol | 7 | 7 |
| Xanthan | 0.35 | 0.35 |
| Iota Carrageenan | 0.6 | 0.6 |
| Sodium Saccharin | 0.5 | 0.5 |
| Tetrasodium Pyrophosphate | 1 | 1 |
| Sodium Tripolyphosphate | 7 | 7 |
| Trisodium Phosphate | 1.1 | 1.1 |
| Gantrez Powder | 1 | 1 |
| Titanium Dioxide | 1 | 1 |
| Abrasive Silica | 12 | 12 |
| High Cleaning Silica | 12 | 12 |
| Flavor | 1.2 | 1.2 |
| Sodium Lauryl Sulfate | 1.5 | 1.5 |
| Stannous Fluoride | 0.454 | ----- |
| plant extract | 0.3 | 0.3 |
| Synthetic Glycerin (99%) | Balance | Balance |
| Color Evaluation* | 5.0 | 5.0 |

| | | |
|---|---|---|
| * Scale: 1: Worst 5: Best | | |

The invention has been described above with respect to various preferred aspects; however it is to be understood the invention is not limited to the disclosed embodiments. Variations and modifications that will occur to the person of skill in the art are also part of the invention, which is defined in the appended claims.

## Claims

1. An oral composition comprising
1) 1-70% by weight of a humectant;
2) 1-70% by weight of at least one abrasive compound;
3) 0.001-5% by weight of a plant extract comprising one or more flavonoid components;
4) an antioxidant selected from stannous compounds, stannate compounds, ammonium sulfates, BHT, and sodium metabisulfite; and
5) less than 6% by weight water.

2. A composition according to claim 1, wherein the plant is selected from rosemary, oregano, green tea, baikal skullcap, *S. lateriflora, S. orthocalyx,* grape seed, and grape skin.

3. A composition according to claim 2, comprising rosemary extract or green tea extract.

4. A composition according to claim 1, comprising a free B-ring flavonoid.

5. A composition according to claim 1, comprising an extract of a *Scutellaria* species.

6. A composition according to claim 1, further comprising an anticalculus system comprising at least one phosphate compound and a anionic carboxylate component.

7. A composition according to claim 1, further comprising an antibacterial component comprising a halogenated diphenylether compound.

8. A composition according to claim 7, wherein the halogenated diphenylether compound comprises triclosan.

9. A method for reducing the extent of discoloration in a toothpaste, the toothpaste being formulated to contain at least one of rosemary extract, green tea extract, oregano extract, baikal skullcap extract, *S. lateriflora* extract, *S. orthocalyx* extact, grape seed extract, and grape skin extract, the extracts containing flavonoid compounds that tend to oxidize and discolor the toothpaste composition over time, the method comprising:
a) formulating the toothpaste to contain less than 10% by weight water; and
b) adding to the toothpaste an amount effective to reduce discoloration of an antioxidant selected form the group consisting of stannous compounds, stannate compounds, ammonium sulfate, phenolic antioxidants, hydroquinone antioxidants, and sodium meta-bisulfite.

10. A method according to claim 9, comprising formulating the toothpaste to contain less than 6% by weight water.

11. A method according to claim 10, comprising adding 0.01-1 % by weight of the antioxidant.

12. A method according to claim 9, wherein the toothpaste comprises rosemary extract or green tea extract.

13. A method according to claim 9, wherein the toothpaste formulation comprises an extract of a *Scutellaria* species.

14. A method according to claim 9, wherein the toothpaste composition comprises an antibacterial component comprising a halogenated diphenylether compound.

15. A method according to claim 14, wherein the halogenated diphenylether compound comprises triclosan.

16. A composition to claim 1, further comprising a zinc ion source.

17. A composition according to claim 16, wherein the zinc ion source is selected from zinc acetate, zinc citrate, zinc gluconate, zinc glycinate, zinc oxide or zinc sulfate.

## Patentansprüche

1. Orale Zusammensetzung, die Folgendes umfasst:
1) 1-70 Gew.-% eines Feuchthaltemittels;
2) 1-70 Gew.-% mindestens einer abrasiven Verbindung;
3) 0,001-5 Gew.-% eines Pflanzenextrakts, der eine oder mehrere Flavonoidkomponenten umfasst;
4) ein Antioxidationsmittel, das aus zinnhaltigen Verbindungen, Stannatverbindungen, Ammoniumsulfaten, BHT und Natriummetabisulfit ausgewählt ist; und
5) weniger als 6 Gew.-% Wasser.

2. Zusammensetzung nach Anspruch 1, wobei die Pflanze aus Rosmarin, Oregano, grünem Tee, Baikal-Helmkraut, S. *lateriflora, S. orthocalyx,* Traubenkern und Traubenschale ausgewählt ist.

3. Zusammensetzung nach Anspruch 2, die Rosmarinextrakt und grünen Teeextrakt umfasst.

4. Zusammensetzung nach Anspruch 1, die ein freies B-Ring-Flavonoid umfasst.

5. Zusammensetzung nach Anspruch 1, die einen Extrakt einer *Scutellaria-Spezies* umfasst.

6. Zusammensetzung nach Anspruch 1, die weiterhin ein gegen Zahnstein wirkendes System umfasst, das mindestens eine Phosphatverbindung und eine anionische Carboxylatkomponente umfasst.

7. Zusammensetzung nach Anspruch 1, die weiterhin eine antibakteriell wirkende Komponente umfasst, die eine halogenierte Diphenyletherverbindung umfasst.

8. Zusammensetzung nach Anspruch 7, wobei die halogenierte Diphenyletherverbindung Triclosan umfasst.

9. Verfahren zur Verringerung des Ausmaßes einer Verfärbung in einer Zahnpasta, wobei die Zahnpasta dahingehend formuliert wird, mindestens einen von Rosmarinextrakt, grünem Teeextrakt, Oreganoextrakt, Baikal-Helmkraut-Extrakt, *S.-lateriflora-Extrakt, S.-orthocalyx-Extrakt,* Traubenkernextrakt und Traubenschalenextrakt zu enthalten, wobei die Extrakte Flavonoidverbindungen enthalten, die dazu neigen, die Zahnpastazusammensetzung im Zeitablauf zu oxidieren und zu verfärben, wobei das Verfahren Folgendes umfasst:
a) Formulieren der Zahnpaste dahingehend, weniger als 10 Gew.-% Wasser zu enthalten; und
b) Zugeben einer Menge eines Antioxidationsmittels, das aus der Gruppe bestehend aus zinnhaltigen Verbindungen, Stannatverbindungen, Ammoniumsulfat, phenolischen Antioxidationsmitteln, Hydrochinon-Antioxidationsmitteln und Natriummetabisulfit ausgewählt ist, zu der Zahnpasta, wobei die Menge dazu wirksam ist, eine Verfärbung zu verringern.

10. Verfahren nach Anspruch 9, das das Formulieren der Zahnpasta dahingehend umfasst, weniger als 6 Gew.-% Wasser zu enthalten.

11. Verfahren nach Anspruch 10, das das Zugeben von 0,01-1 Gew.-% des Antioxidationsmittels umfasst.

12. Verfahren nach Anspruch 9, wobei die Zahnpasta Rosmarinextrakt oder grünen Teeextrakt umfasst.

13. Verfahren nach Anspruch 9, wobei die Zahnpastaformulierung einen Extrakt einer *Scutellaria-*Spezies umfasst.

14. Verfahren nach Anspruch 9, wobei die Zahnpastazusammensetzung eine antibakteriell wirkende Komponente umfasst, die eine halogenierte Diphenyletherverbindung umfasst.

15. Verfahren nach Anspruch 14, wobei die halogenierte Diphenyletherverbindung Triclosan umfasst.

16. Zusammensetzung nach Anspruch 1, die weiterhin eine Zinkionenquelle umfasst.

17. Zusammensetzung nach Anspruch 16, wobei die Zinkionenquelle aus Zinkacetat, Zinkcitrat, Zinkgluconat, Zinkglycinat, Zinkoxid oder Zinksulfat ausgewählt ist.

## Revendications

1. Une composition buccale comprenant :
1) 1 à 70 % en poids d'un humidifiant ;
2) 1 à 70 % en poids d'au moins un composé abrasif ;
3) 0,001 à 5 % en poids d'un extrait de plante comprenant un ou plusieurs composants flavonoïdes ;
4) un antioxydant sélectionné dans le groupe constitué par des composés stanneux, des composés de stannate, des sulfates d'ammonium, le BHT et le métabisulfite de sodium ; et
5) moins de 6 % en poids d'eau.

2. Une composition selon la revendication 1, dans laquelle la plante est sélectionnée parmi le romarin, l'origan, le thé vert, la calotte de Baïkal, le *S. lateriflora,* le *S. orthocalyx*, le pépin de raisin et la peau de raisin.

3. Une composition selon la revendication 2, comprenant un extrait de romarin ou un extrait de thé vert.

4. Une composition selon la revendication 1, comprenant un flavonoïde à cycle B libre.

5. Une composition selon la revendication 1, comprenant un extrait d'une espèce de *Scutellaria.*

6. Une composition selon la revendication 1, comprenant en outre un système antitartre comprenant au moins un composé de phosphate et un composant de carboxylate anionique.

7. Une composition selon la revendication 1, comprenant en outre un composant antibactérien comprenant un composé de diphényléther halogéné.

8. Une composition selon la revendication 7, dans laquelle le composé de diphényléther halogéné comprend le triclosan.

9. Un procédé pour réduire l'étendue de la décoloration dans un dentifrice, le dentifrice étant formulé pour contenir au moins un extrait parmi un extrait de romarin, un extrait de thé vert, un extrait d'origan, un extrait de calotte de Baïkal, un extrait de *S. lateriflora,* un extrait de *S. orthocalyx*, un extrait de pépin de raisin et un extrait de peau de raisin, les extraits contenant des composants flavonoïdes qui ont tendance à s'oxyder et à décolorer la composition de dentifrice dans le temps, le procédé consistant à :
a) formuler le dentifrice pour contenir moins de 10 % en poids d'eau ; et
b) ajouter au dentifrice une quantité efficace pour réduire la décoloration d'un antioxydant sélectionné dans le groupe constitué par les composés stanneux, les composés de stannate, le sulfate d'ammonium, les antioxydants phénoliques, les antioxydants d'hydroquinone et le métabisulfite de sodium.

10. Un procédé selon la revendication 9, consistant à formuler le dentifrice pour contenir moins de 6 % en poids d'eau.

11. Un procédé selon la revendication 10, consistant à ajouter 0,01 à 1 % en poids de l'antioxydant.

12. Un procédé selon la revendication 9, dans lequel le dentifrice comprend un extrait de romarin ou un extrait de thé vert.

13. Un procédé selon la revendication 9, dans lequel la formulation du dentifrice comprend un extrait d'une espèce de *Scutellaria*.

14. Un procédé selon la revendication 9, dans lequel la composition de dentifrice comprend un composant antibactérien comprenant un composé de diphényléther halogéné.

15. Un procédé selon la revendication 14, dans lequel le composé de diphényléther halogéné comprend le triclosan.

16. Une composition selon la revendication 1, comprenant en outre une source d'ions de zinc.

17. Une composition selon la revendication 16, dans laquelle la source d'ions de zinc est sélectionnée parmi l'acétate de zinc, le citrate de zinc, le gluconate de zinc, le glycinate de zinc, l'oxyde de zinc ou le sulfate de zinc.
